(19) Europäisches Patentamt / European Patent Office / Office européen des brevets

(11) **EP 4 470 610 A1**

(12) **DEMANDE DE BREVET EUROPEEN**

(43) Date de publication:
**04.12.2024  Bulletin 2024/49**

(21) Numéro de dépôt: **24179182.1**

(22) Date de dépôt: **31.05.2024**

(51) Classification Internationale des Brevets (IPC):
**A61P 17/00** *(2006.01)*  **A61K 8/9789** *(2017.01)*
**A61K 36/31** *(2006.01)*

(52) Classification Coopérative des Brevets (CPC):
(C-Sets disponibles)
**A61P 17/00; A61K 8/00; A61K 8/9789;
A61K 33/00; A61K 36/31**  (Cont.)

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH MA MD TN**

(30) Priorité: **31.05.2023  FR 2305400**

(71) Demandeur: **Societe Industrielle Limousine d'Application Biologique
19240 Saint Viance (FR)**

(72) Inventeur: **PAUFIQUE, Jean
19130 OBJAT (FR)**

(74) Mandataire: **Aquinov
12, Cours Xavier Arnozan
33000 Bordeaux (FR)**

(54) **EXTRAIT DE NASTURTIUM OFFICINALE ET SES UTILISATIONS**

(57)  La présente invention concerne un principe actif obtenu à partir d'une matière première **végétale** de l'espèce *Nasturtium officinale* et ses utilisations, notamment pour lutter contre le stress.

**EP 4 470 610 A1**

(52) Classification Coopérative des Brevets (CPC):
(Cont.)

C-Sets
**A61K 36/31, A61K 2300/00**

**Description**

**Domaine technique**

L'invention concerne un principe actif obtenu à partir d'une matière première végétale de l'espèce *Nasturtium officinale* et ses utilisations, en particulier pour lutter contre le stress, notamment ses effets délétères sur la santé.

**Etat de l'art**

Le stress psychologique se définit comme l'ensemble des réactions psychologiques et physiques de l'organisme lui permettant de surmonter rapidement une situation dangereuse, inattendue ou inquiétante. Si le stress a permis à nos ancêtres d'échapper aux griffes des prédateurs et a ainsi incontestablement participé à la survie de l'espèce, il s'installe aujourd'hui de manière chronique dans notre quotidien avec des conséquences délétères sur la santé. Le stress est ainsi considéré comme un véritable fléau et probablement le mal du siècle. Récemment, la crise du COVID-19 a participé à l'augmentation du niveau d'anxiété générale, sentiment dû à la perception d'une menace potentielle pour la santé et l'avenir de la société.

Au niveau cutané, le stress a d'une part, un impact comportemental important se traduisant par une crispation des muscles faciaux, à l'origine de la formation prématurée de rides d'expression caractéristiques. Afin de gommer virtuellement les effets néfastes du stress sur la peau, l'utilisation de filtres de retouches photos à destination des réseaux sociaux est devenue une pratique courante. Toutefois, cette pratique est susceptible d'accentuer le souhait des consommateurs, souvent jeunes, à ressembler physiquement à des selfies retouchés et ainsi conduire à une baisse de l'estime de soi.

D'autre part, le stress possède également des effets cutanés systémiques. En effet, la peau est le siège de sécrétions de neuromédiateurs du stress, dont les conséquences se traduisent par une altération de la fonction barrière et une diminution de l'éclat du teint.

Il existe donc un besoin pour un principe actif permettant de lutter contre le stress, mais également contre ses effets indésirables.

Dans ce contexte, des études scientifiques ont démontré le lien biologique intime existant entre le stress psychologique et la peau. En effet, les cellules cutanées et les neurones possèdent de nombreuses similitudes et connexions. La peau est richement innervée par un réseau de fibres sensitives transmettant au cerveau de nombreux stimuli perçus tels que le toucher, la température ou encore la douleur. Il a ainsi été mis en évidence l'existence d'une communication paracrine spécifique et rapide des kératinocytes vers les neurones sensoriels par l'intermédiaire de médiateurs et de récepteurs identiques, notamment à travers les voies de l'ocytocine et des endocannabinoïdes.

L'ocytocine, également surnommée « hormone du bonheur », est décrite pour son implication majeure dans la réduction du niveau de stress en procurant un effet d'apaisement et de bien-être. Ce neuromédiateur est libéré par les kératinocytes suite à l'activation du mécanorécepteur PIEZO1 par des stimulations tactiles douces, favorisant ainsi leur prolifération. Aussi, il est déjà connu des approches destinées à apaiser le corps et l'esprit, basées sur la relaxation, la méditation ou encore la sophrologie, afin de contrer les effets systémiques du stress.

Les récepteurs endocannabinoïdes sont également impliqués dans la régulation des mécanismes induits par le stress. Au niveau cutané, une période de stress psychologique aigue active la signalisation associée au récepteur CB1, ce qui permet de rétablir une fonction barrière effective.

Aussi, l'inventeur s'est intéressé à un grand nombre de matières premières végétales susceptible de pouvoir agir sur le stress et ses conséquences. Il peut s'agir de conséquences comportementales du stress, par exemple l'apparition de rides d'expression, mais également de conséquences systémiques du stress, par exemple l'irritabilité, les troubles du sommeil, et l'altération de la fonction barrière et des paramètres de l'éclat du teint.

Au cours de ses travaux de recherche, l'inventeur a ainsi identifié un extrait de cresson de fontaine et démontré son efficacité sur les médiateurs du bien-être, la contraction musculaire, mais également son efficacité sur les cellules cutanées.

Ainsi, l'inventeur s'est particulièrement intéressé à l'espèce végétale *Nasturtium officinale* pour développer un nouvel ingrédient dérivé du naturel pour agir et lutter contre le stress. Il est connu des produits à base de *Nasturtium officinale,* notamment des produits cosmétiques ayant des effets hydratant et anti-rides. Toutefois, aucun de ces produits ne décrit une efficacité liée au stress, en particulier aucun effet sur les récepteurs et médiateurs du « bien-être » n'a été rapporté. C'est l'objet de la présente invention.

**Résumé de l'invention**

Ainsi, l'invention concerne un extrait de *Nasturtium officinale,* en particulier un principe actif comprenant ledit extrait de *Nasturtium officinale* pour son utilisation cutanée pour lutter contre le stress.

En effet, le principe actif selon l'invention procure un effet « bien-être » en favorisant l'expression des médiateurs et récepteurs de la réponse au stress, permettant ainsi de lutter contre le stress. En outre, il présente une action myorelaxante en agissant sur la jonction neuro-musculaire. Cette double action spécifique neutralise les effets délétères du stress sur la peau et stimule également la production de l'hormone du bonheur, diminuant alors le niveau de stress chez un individu. Par conséquent, l'individu est plus détendu, apaisé ; la peau apparait également plus détendue, améliorant ainsi sa qualité. Ainsi, l'invention vise un principe actif comprenant l'extrait de *Nasturtium officinale* pour son utilisation pour lutter contre le stress, en application topique.

Préférentiellement, le principe actif selon l'invention est destiné à être utilisé pour lutter contre les effets cutanés du stress. En agissant sur les effets cutanés du stress, le principe actif selon l'invention permet de lutter contre l'impact comportemental et systémique du stress.

Selon un objet, le principe actif selon l'invention présente préférentiellement un effet myorelaxant cutané, et par conséquent un effet lissant. De façon particulièrement surprenante, cet effet lissant est immédiat. Une amélioration significative de la neurostimulation est observée dès 6h après application du principe actif selon l'invention. Ainsi, le principe actif selon l'invention stimule également les médiateurs et récepteurs du bien-être.

En outre, l'invention a également pour objet préféré le principe actif selon l'invention pour son utilisation pour :

- augmenter la sécrétion d'ocytocine cutanée (OXT), et/ou
- augmenter l'expression du récepteur de type canal ionique PIEZO1, et/ou
- augmenter l'expression du récepteur à l'ocytocine OXTR, et/ou
- augmenter la synthèse des récepteurs aux cannabinoïdes, préférentiellement le récepteur aux cannabinoïdes de type 1 (CB1R).

Selon un autre mode de réalisation préféré, l'invention a également pour objet le principe actif selon l'invention pour son utilisation pour améliorer l'éclat du teint, à savoir l'utilisation cosmétique dudit principe actif pour améliorer l'éclat du teint.

Le principe actif selon l'invention est un extrait de *Nasturtium officinale,* également connu sous la dénomination cresson de fontaine.

Préférentiellement, l'extrait de *Nasturtium officinale* est un hydrolysat de *Nasturtium officinale,* plus préférentiellement, l'hydrolysat de *Nasturtium officinale* est un hydrolysat enzymatique de *Nasturtium officinale.*

Selon un autre objet préféré de l'invention, l'hydrolysat de *Nasturtium officinale* comprend des minéraux. Plus préférentiellement, les cendres minérales représentent au moins 25% en poids du poids total de l'extrait.

Selon un autre objet préféré, l'extrait comprend au moins un minéral, encore plus préférentiellement un mélange de plusieurs minéraux. Ainsi, l'extrait comprend préférentiellement au moins un minéral choisi parmi les chlorures, le soufre, le sodium, le potassium, le magnésium, le phosphore, le calcium, et leurs combinaisons.

Enfin, selon un dernier aspect, l'invention a également pour objet une utilisation, notamment cosmétique, du principe actif comprenant au moins un extrait de *Nasturtium officinale,* selon l'un des quelconques objets décrits précédemment, pour lutter contre les effets cutanés liés au stress et à l'anxiété. Ainsi, l'invention se rapporte également à une utilisation cosmétique du principe actif selon l'invention pour améliorer l'éclat du teint et/ou l'effet lissant.

D'autres caractéristiques et avantages ressortiront de la description détaillée de l'invention, des exemples et des figures qui vont suivre.

**Description détaillée de l'invention**

Définition

Par « extrait de *Nasturtium officinale* » au sens de la présente invention, on entend au moins une molécule ou toute molécule ou mélange d'au moins deux molécules obtenu(e) à partir d'une matière première, à savoir le cresson de fontaine, en particulier l'espèce *Nasturtium officinale,* quel que soit le procédé d'extraction de ladite ou desdites molécule(s). Il peut s'agir par exemple d'un extrait obtenu par extraction aqueuse et/ou hydroalcoolique et/ou hydroglycolique et/ou obtenu après au moins une étape d'hydrolyse, par exemple enzymatique ou acide. Ainsi, au sens de l'invention un tel extrait est un ingrédient dérivé du naturel obtenu par des procédés chimiques et/ou biologiques spécifiques et définis visant à le modifier chimiquement, modifications qui sont intentionnelles, et qui permettent d'obtenir des structures de molécules qui ne sont donc plus toutes identiques aux molécules présentes dans la nature. Très préférentiellement, l'extrait de *Nasturtium officinale* est un hydrolysat de *Nasturtium officinale.*

Par « hydrolysat de *Nasturtium officinale* » au sens de l'invention, on entend un extrait issu de l'espèce végétale *Nasturtium officinale,* obtenu par un procédé comprenant au moins une étape d'hydrolyse de *Nasturtium officinale.* Le terme hydrolysat de *Nasturtium officinale* exclut les procédés de fermentation de *Nasturtium officinale* par un microorganisme ou une infusion/décoction/macération de *Nasturtium officinale.*

Par « principe actif » au sens de l'invention, on entend un extrait comprenant au moins une molécule, préférentiellement un ensemble de molécules présentant un effet sur le stress, en particulier pour lutter contre les effets cutanés du stress.

Par « lutter contre les effets cutanés du stress » au sens de l'invention, on entend une action visant à stimuler la synthèse et l'expression des médiateurs et récepteurs du bien-être (notamment OXT, PIEZO1, OXTR, CB1R) au niveau des cellules cutanées et/ou présentant une action myorelaxante, par exemple en agissant sur la jonction neuromusculaire.

Principe actif selon l'invention

La présente invention a donc pour objet un principe actif comprenant au moins un extrait de *Nasturtium officinale.* Le cresson de fontaine, *Nasturtium officinale,* est une espèce de plante vivace de la famille des *Brassicaceae,* connu depuis l'Antiquité pour ses qualités alimentaires et médicinales ainsi que sa richesse en éléments minéraux. Il est également connu pour être en riche en flavonoïdes, vitamines et glucosinolates.

Dans ce contexte, l'inventeur s'est intéressé à cette matière première végétale et a développé un principe actif à base d'un extrait de *Nasturtium officinale,* permettant de lutter contre le stress en agissant notamment sur les médiateurs et les récepteurs du bien-être. Le principe actif selon l'invention favorise ainsi avantageusement l'expression des médiateurs et récepteurs de la réponse au stress et présente, en outre, une action myorelaxante en agissant sur la jonction neuro-musculaire.

Ainsi, l'invention se rapporte à un extrait de *Nasturtium officinale,* en particulier un principe actif comprenant ledit extrait de *Nasturtium officinale* pour son utilisation pour lutter contre le stress, en application topique.

L'accès (selon la définition du Protocole de Nagoya) à la matière première végétale (*Nasturtium officinale*) a été effectué conformément à la règlementation nationale APA (Accès et Partage des Avantages) du pays d'origine (France).

L'inventeur a caractérisé les molécules constituant l'extrait selon l'invention et a notamment déterminé la teneur en minéraux, par la pesée des résidus issus de l'incinération à 550°C dans un four à moufle électrique permettant d'obtenir la teneur en cendres minérales. Les teneurs en sucres totaux et en protéines ont été déterminées, respectivement selon la méthode de DUBOIS (Dubois M. et al., Analytical chemistry, 28, 3, 350-356, 1956) et la méthode au BCA (Bincinchoninic acid ; P. K. Smith et al. Measurement of protein using bicinchoninic acid, Anal. Biochem. 150, 1, 76, 1985).

Ainsi, le principe actif selon l'invention comprend préférentiellement au moins des minéraux et/ou des sucres, et/ou des protéines.

L'inventeur a caractérisé la nature de la fraction minérale de l'extrait selon l'invention. Ainsi, selon un objet de l'invention, l'extrait de *Nasturtium officinale* comprend des cendres minérales, celles-ci représentent avantageusement au moins 25% en poids du poids total de l'extrait. Préférentiellement, l'extrait comprend au moins un minéral choisi parmi les chlorures, le sodium, le potassium, le soufre, le calcium, le magnésium, le phosphore, et leurs combinaisons.

Selon un objet de l'invention, les protéines présentes dans l'extrait composant le principe actif selon l'invention ont préférentiellement une masse molaire inférieure à 2 000 Da.

Lorsque l'extrait comprend des sucres, l'extrait comprend majoritairement des monosaccharides et des oligosaccharides de masses molaires comprises entre 360 Da et 1260 Da. Avantageusement, l'extrait selon l'invention comprend au moins un sucre choisi parmi le glucose, l'acide galacturonique, le fructose, le galactose, le xylose, le mannose, l'arabinose, et leurs combinaisons.

Pour améliorer le rendement de production du principe actif, au moins une hydrolyse est mise en oeuvre. Ainsi, selon un objet particulièrement d'intérêt, l'extrait de *Nasturtium officinale* est obtenu à partir d'une hydrolyse, plus préférentiellement une hydrolyse enzymatique. Avantageusement, l'extrait de *Nasturtium officinale* est un hydrolysat de *Nasturtium officinale.*

Plus préférentiellement, l'hydrolysat de *Nasturtium officinale* est un hydrolysat enzymatique de *Nasturtium officinale.*

Selon un objet particulièrement préféré de l'invention, l'extrait est susceptible d'être obtenu par un procédé de préparation comprenant les étapes suivantes :

    a. Solubilisation de *Nasturtium officinale,* dans l'eau a raison d'au moins 50 g/L, préférentiellement des parties aériennes de *Nasturtium officinale*
    b. Extraction par hydrolyse, préférentiellement hydrolyses enzymatiques
    c. Séparation des phases soluble et insoluble, préférentiellement par décantation, filtration ou centrifugation,
    d. Traitement thermique,
    e. Concentration de la phase soluble, préférentiellement par filtration, atomisation, ou lyophilisation.
    f. Éventuellement, désodorisation et/ou décoloration,
    g. Éventuellement, filtration et filtration stérilisante

Le principe actif cosmétique selon l'invention comprend avantageusement au moins un excipient choisi parmi un conservateur, un antioxydant, un stabilisant, un support d'atomisation et/ou leur combinaison.

L'extrait selon l'invention présent dans le principe actif cosmétique peut se présenter sous forme liquide ou sous forme solide ou sous forme de film.

Lorsqu'il se présente sous forme liquide, le principe actif selon l'invention est exclusivement constitué par l'extrait de *Nasturtium officinale* accompagné de stabilisant et/ou système de conservation.

L'extrait sous forme liquide se présente préférentiellement sous forme d'une solution aqueuse liquide limpide, avec une faible odeur et une couleur jaune clair à jaune. Il peut toutefois être moins coloré.

La teneur en matières sèches de l'extrait peut être déterminée par la pesée des résidus issus du séchage de l'extrait selon l'invention à 105°C dans une étuve jusqu'à l'obtention d'un poids constant. Préférentiellement, l'extrait selon l'invention sous forme liquide a une teneur en matières sèches de 20 g/L à 100 g/L, préférentiellement de 40 g/L à 60 g/L.

Lorsqu'il se présente sous forme solide, le principe actif selon l'invention est préférentiellement constitué par l'extrait de *Nasturtium officinale* tel que précédemment décrit et par un support choisi parmi la maltodextrine, la gomme arabique ou la lécithine de soja. Selon un mode de réalisation, particulièrement adapté, l'extrait représente au moins 10% en poids du principe actif et le support au plus 90% en poids du principe actif.

Dans le cas d'une forme solide dans laquelle le principe actif est associé à un support, les teneurs en minéraux, en protéines et en sucres dans le principe actif sont modifiées, le support étant généralement constitué majoritairement de sucres.

Le principe actif selon l'invention peut aussi être présenté sous forme d'un film tel que décrit dans le brevet FR3079145. Dans ce cas, l'extrait de *Nasturtium officinale* représente au moins 0,1% en poids du film.

Lorsqu'il se présente sous forme de film, le principe actif comprend :

- au moins l'extrait de *Nasturtium officinale* selon l'invention.
- au moins une charge minérale, et
- au moins un polymère d'origine naturelle, et
- au moins un plastifiant, et
- au moins un tensio-actif.

Le polymère d'origine naturelle peut être choisi parmi la : Pectine, Gomme tamarin, Alginate, Pullulane, Psyllium, Xanthane, Guar, Tara, Caroube, Agar, Gomme arabique, Gellane, Dextran, Carraghénane, Cellulose, Konjac et le Chitosan.

Le plastifiant peut être choisi parmi : Glycérol, Sorbitol, Saccharose, Erythritol, Urée, Propylène glycol et le Butylène glycol.

La charge minérale peut être choisie parmi : Carbonate de calcium, argile verte, kaolin, perlite, talc, silicate de magnésium, mica, sericite diatomée, silice, sulfate de calcium, chlorure de calcium, chlorure de potassium, oxyde de fer et l'oxyde de zinc.

Le principe actif peut comprendre, en outre, un pigment pour colorer le film.

Composition selon l'invention

Le principe actif selon l'invention peut éventuellement être intégré dans une composition, notamment une composition comprenant au moins 0,1% en poids dudit principe actif et un milieu physiologiquement acceptable, préférentiellement un milieu cosmétiquement acceptable.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux, poudres, ou fond de teint ou sous forme de film. Elles peuvent être plus ou moins fluides et avoir l'aspect de crèmes, émulsions, gels, masques ou tous autres aspects connus de l'Homme du métier.

Préférentiellement, il peut s'agir de compositions comprenant entre 0,1 et 20% du principe actif liquide selon l'invention, plus préférentiellement entre 0,5 et 10%.

Ces compositions comprennent, outre le principe actif, un milieu physiologiquement acceptable tel qu'un milieu cosmétiquement acceptable, c'est-à-dire qui ne provoque pas de sensations d'inconfort pour l'utilisateur telles que des rougeurs, tiraillements ou picotements.

Les compositions selon l'invention peuvent également contenir comme ingrédient au moins un composé choisi parmi :

- les huiles, qui peuvent être choisies notamment parmi les huiles de silicone, linéaires ou cycliques, volatiles ou non volatiles,

- les cires, telles que l'ozokérite, la cire de polyéthylène, la cire d'abeille ou la cire de carnauba,
- les élastomères de silicone,
- les tensioactifs, de préférence émulsionnants, qu'ils soient non ioniques, anioniques, cationiques ou amphotères,
- les co-tensioactifs, tels que les alcools gras linéaires,
- les épaississants et/ou gélifiants,
- les humectants, tels que les polyols comme la glycérine,
- les colorants, les conservateurs, les charges,
- les tenseurs,
- les séquestrants,
- les parfums,
- et leurs mélanges, sans que cette liste soit limitative.

Des exemples de tels ingrédients sont cités notamment dans le Dictionnaire CTFA (International Cosmetic ingrédient Dictionary and Handbook publié par le Personal Care Product Council).

Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Procédé de préparation de l'extrait selon l'invention

L'extrait constituant ou contenu dans le principe actif selon l'invention peut être obtenu par tout moyen.

Le cresson de fontaine est une plante à croissance rapide qui ne nécessite que peu de fertilisants. Avantageusement, la culture, est réalisée dans des cressonnières, par semis successifs.

Selon un mode de réalisation préféré, le procédé d'extraction comprend au moins une étape de solubilisation de *Nasturtium officinale,* préférentiellement des parties aériennes de *Nasturtium officinale,* une étape d'hydrolyse, préférentiellement au moins une hydrolyse enzymatique et une étape de filtration.

Selon un mode de réalisation particulièrement adapté, le principe actif selon l'invention est obtenu par la mise en oeuvre des étapes suivantes :

a. Solubilisation de parties aériennes de *Nasturtium officinale,* dans l'eau à raison d'au moins 50 g/L,
b. Hydrolyse, préférentiellement hydrolyses enzymatiques,
c. Séparation des phases soluble et insoluble,
d. Traitement thermique,
e. Concentration de la phase soluble, préférentiellement par tri moléculaire,
f. Éventuellement, désodorisation et/ou décoloration,
g. Éventuellement, filtration et filtration stérilisante.

L'étape de traitement thermique est avantageusement réalisée pour inactiver la ou les enzyme(s) présente(s). Cette inactivation est alors réalisée selon les préconisations du fournisseur de la ou des enzyme(s) utilisée(s).

La séparation de la phase soluble et insoluble est réalisée par tout moyen connu de l'homme du métier, par exemple par centrifugation, filtration ou décantation. La séparation des phases soluble et insoluble est réalisée pour récupérer la phase soluble.

L'étape de tri moléculaire permet la concentration/purification de la phase soluble récupérée. Elle est réalisée afin de sélectionner plus spécifiquement les molécules actives.

L'extrait obtenu après hydrolyse et concentration, avant ou après concentration et filtration stérilisante, est un extrait de *Nasturtium officinale,* et constitue une première forme du principe actif selon l'invention, se présentant alors sous forme liquide.

L'extrait obtenu peut ensuite être séché et associé ou non à un support, pour se présenter sous forme solide. Cette phase peut être réalisée par la mise en oeuvre des étapes suivantes :

- un support d'atomisation, de préférence la maltodextrine, est ajouté dans l'extrait de *Nasturtium officinale,* d'au plus 90% (en masse/volume) ;
- cette solution est ensuite concentrée sous vide ;
- un traitement antimicrobien peut être réalisé ;
- l'atomisation permet d'obtenir une poudre.

L'extrait obtenu peut également être utilisé pour être présenté sous forme d'un film. Cette phase peut être réalisée par la mise en oeuvre des étapes suivantes :

- Formulation sous forme liquide des différents composants : extrait de *Nasturtium officinale* selon l'invention, charge minérale, polymère d'origine naturelle, plastifiant, tensio-actif,
- Dépôt de la formule sur un support,
- Evaporation de l'eau dans un four chauffé à une température comprise entre 70 et 100°C,
- Découpe au laser selon la forme voulue.

Les étapes des procédés décrits ci-avant, prises individuellement sont usuelles dans le domaine des extractions d'actifs à partir de matières premières naturelles et l'homme du métier est à même d'en ajuster les paramètres réactionnels sur la base de ses connaissances générales.

Utilisation du principe actif selon l'invention

Enfin, comme évoqué précédemment, l'inventeur s'est particulièrement intéressé à cette espèce végétale pour développer un principe actif pour agir sur le stress et ses effets délétères.

Ainsi, l'invention concerne un extrait de *Nasturtium officinale,* en particulier un principe actif comprenant ledit extrait de *Nasturtium officinale,* selon l'un des quelconques objets précédemment décrits, pour lutter contre le stress. Préférentiellement, ledit principe actif est destiné à être appliqué par la voie cutanée.

En effet, de façon surprenante, l'inventeur a observé que le principe actif selon l'invention permet de lutter contre le stress ainsi que ses conséquences, sur les cellules cutanées. Ainsi, l'invention se rapporte préférentiellement, au principe actif de l'invention pour lutter contre les effets cutanés liés au stress, en présentant une action myorelaxante par l'intermédiaire de la jonction neuro-musculaire. En outre, il agit également en stimulant les médiateurs du bien-être, notamment en agissant sur la voie de l'ocytocine et la voie des endocannabinoïdes, permettant de lutter contre le stress.

En effet, lors de ses travaux de recherche, l'inventeur a observé que le principe actif selon l'invention a une efficacité sur les acteurs de la communication paracrine entre les kératinocytes et les terminaisons nerveuses. Le système ocytocine et la signalisation cellulaire liée au récepteur CB1R ont alors été étudiés afin de démontrer que le principe actif selon l'invention stimule les médiateurs et récepteurs du bien-être via la stimulation de la sécrétion d'ocytocine (OXT), ainsi que l'induction de l'expression des récepteurs PIEZO1 et OXTR et CB1R.

L'ocytocine est un neuropeptide synthétisé au niveau de l'hypothalamus en réponse à un sentiment de bien-être mais également en réponse au toucher. Cette hormone dite du bonheur joue un rôle important dans les interactions sociales en régulant notamment le sentiment d'anxiété, l'amitié, l'attachement à autrui ou encore l'empathie. A l'échelle cutanée, l'ocytocine (OXT) est sécrétée au niveau de l'épiderme et du derme. Son récepteur (OXTR), également exprimé au niveau cutané, appartient à la famille des récepteurs couplés aux protéines G. Il permet, suite à la fixation de l'ocytocine, d'activer des voies de signalisation intracellulaires. Ce récepteur est impliqué dans la médiation du bien-être et son activation dépend notamment de minéraux.

Le mécanorécepteur PIEZO1 est un canal ionique exprimé par les kératinocytes et les terminaisons nerveuses. Il permet de stimuler les fibres nerveuses sensorielles et transmettre la sensation de toucher de la peau. Récemment, il a été montré que l'activation de PIEZO1 par des stimulations tactiles douces permet notamment d'induire la sécrétion d'ocytocine par les kératinocytes.

La voie des endocannabinoïdes est également bien connue pour son effet bénéfique tant au niveau mental qu'au niveau des tissus périphériques comme la peau. Le récepteur aux cannabinoïdes de type 1 (CB1R) est un récepteur membranaire couplé aux protéines G dont la signalisation cellulaire est dépendante des minéraux. Exprimé au niveau cutané, ce récepteur participe au maintien de l'homéostasie épidermique et renforce la fonction barrière.

Ainsi, le principe actif selon l'invention présente une action sur les voies de l'ocytocine et des endocannabinoïdes cutanées, par l'intermédiaire de la stimulation de l'ocytocine (OXT), des récepteurs PIEZO1, OXTR, mais également du récepteur CB1R, voies bien connues pour être impliquées dans le bien-être et la lutte contre le stress. De façon particulièrement, surprenante, l'effet est immédiat avec une amélioration de la neurostimulation, dès 6 heures après application.

Ainsi, l'invention a avantageusement pour objet, le principe actif pour son utilisation :

- pour augmenter la sécrétion d'ocytocine cutanée (OXT), et/ou
- pour augmenter l'expression du récepteur PIEZO1 et/ou
- pour augmenter l'expression du récepteur OXTR, et/ou
- pour augmenter la synthèse du récepteur aux cannabinoïdes de type 1 (CB1R).

Ainsi, le principe actif stimule la production de l'hormone du bonheur : l'ocytocine, réduisant le niveau de stress. En outre, il améliore la qualité de la peau, l'éclat du teint est alors sublimé.

Selon un autre objet, le principe actif selon l'invention présente également une action myorelaxante et diminue la contraction musculaire. En effet, au niveau moléculaire, la contraction des muscles du visage résulte de la stimulation de motoneurones et de la libération du messager de la contraction musculaire, l'acétylcholine, au niveau des synapses neuromusculaire. En réponse à un influx nerveux, des échanges minéraux se mettent en place et conduisent à la fusion de vésicules d'acétylcholine contenues dans la terminaison nerveuse au niveau de la membrane pré-synaptique. Une fois libérée dans la fente synaptique, l'acétylcholine se fixe au niveau des récepteurs spécifiques localisés à la surface de la fibre musculaire. Des échanges minéraux interviennent et induisent le déclenchement d'une contraction musculaire.

Le principe actif selon l'invention présente un effet myorelaxant. Cette efficacité sur la jonction neuro-musculaire se traduit par un effet lissant, préférentiellement un effet lissant immédiat. En effet, l'inventeur a observé un effet lissant dès 5 jours après application. Ainsi, l'invention se rapporte également à l'utilisation du principe actif selon l'invention pour un effet myorelaxant cutané et/ou un effet lissant. Préférentiellement l'effet lissant est immédiat. Cette efficacité se traduit également par un effet « soft focus » du principe actif selon l'invention. L'effet perçu par les volontaires est similaire à celui obtenu avec un filtre de retouche photo.

L'invention est à présent illustrée par des exemples non limitatifs de compositions selon l'invention et par des résultats.

**Exemples**

Exemple 1 : Principe actif selon l'invention

Le principe actif de l'exemple 1 est obtenu à partir de l'espèce végétale *Nasturtium officinale.* Le principe actif de l'exemple 1 est obtenu par le procédé suivant :

    a. Solubilisation de parties aériennes de *Nasturtium officinale,* dans l'eau a raison d'au moins 50 g/L,
    b. Hydrolyses enzymatiques,
    c. Séparation des phases soluble et insoluble,
    d. Traitement thermique, et
    e. Concentration de la phase soluble.

Le principe actif selon l'exemple 1 est constitué d'un extrait de *Nasturtium officinale* comprenant au moins 25% de minéraux.

Le principe actif obtenu possède notamment les caractéristiques suivantes :

- Teneur en Matières Sèches = 52,8 g/L, dont :
- Teneur en cendres minérales = 23 g/L soit 44 % en poids par rapport à la matière sèche
- Teneur en Sucres Totaux = 13,8 g/L soit 26 %, en poids par rapport à la matière sèche
- Teneur en protéines = 9 %, en poids par rapport à la matière sèche

Exemple 2 : Composition selon l'invention

Un exemple de formulation comprenant le principe actif selon l'invention sous forme d'un gel-crème est présenté dans le tableau 1 ci-après.

[Tableau 1]

|   | Ingrédients | % |
|---|---|---|
| A | Eau Purifiée | qsp 100 |
|   | Glycereth-26 | 3,00 |
| B | Sodium Acrylate/Sodium Acryloyldimethyl Taurate Copolymer & C15-19 Alkane & Polyglyceryl-6 Laurate & Polyglycerin-6 | 1,50 |
|   | Coco-Caprylate | 4,50 |
|   | Triheptanoin | 3,50 |
|   | Cetearyl Ethylhexanoate | 2,50 |
|   | Acrylates/C10-30 Alkyl Acrylate Crosspolymer | 0,30 |
| C | Solution Soude 28% | qsp pH |

(suite)

| | | Ingrédients | % |
|---|---|---|---|
| | D | Butylène Glycol | 5,00 |
| | | Boron Nitride | 0,75 |
| | **E** | **Principe Actif Invention** | **2,00** |

La composition selon l'exemple 2 peut notamment être obtenue par le procédé suivant :

- Ajouter B dans A sous agitation vive. Agiter pendant 20 minutes.
- Ajuster le pH avec C à 6,0 - 6,5.
- Sous agitation modérée, ajouter le pré-mélange D puis E.

La composition est alors sous forme d'un gel-crème épais, blanc et brillant, présentant un pH de 6,3 et une viscosité (C/5rpm) égale à 35 000cP.

Exemple 3 : Composition selon l'invention

Un exemple de formulation comprenant le principe actif selon l'invention sous forme d'un masque bien-être à la texture gel crème fraîche est présenté dans le tableau 2 suivant.

[Tableau 2]

| | Ingrédients | % |
|---|---|---|
| A1 | Eau Purifiée | qsp 100 |
| | Conservateur | qs |
| | Butylène Glycol | 3,00 |
| A2 | Glycérine | 2,00 |
| | Sclerotium Gum & Xanthan Gum | 0,20 |
| B | Sodium Stearoyl Glutamate | 0,25 |
| | Pentaerythrityl Distearate | 1,50 |
| | Isocetyl Stearoyl Stéarate | 3,00 |
| | Cetearyl Ethylhexanoate | 5,00 |
| | Diisopropyl Adipate | 4,00 |
| | Dimethicone | 3,00 |
| C | Hydroxyethyl Acrylate/Sodium Acryloyldimethyl Taurate Copolymer & Polyisobutene & PEG-7 Trimethylolpropane Coconut Ether | 2,00 |
| **D** | **Principe Actif Invention** | **2,00** |
| E | Solution Acide Citrique 10% | qsp pH |

La composition de l'exemple 3 peut notamment être obtenue par le procédé suivant :

- Ajouter A2 dans A1 sous agitation et chauffer à 80°C.
- Placer B sous agitation et chauffer à 80°C.
- Emulsionner B dans A sous agitation cisaillante pendant 10 minutes.
- A température ambiante, sous agitation douce, ajouter C puis D.
- Ajuster le pH à 5,0 - 5,5 avec E.

La composition est alors sous forme d'un masque ayant la texture d'un gel-crème épais, blanc, présentant un pH de 5,3 et une viscosité (C/5rpm) égale à 58 000cP.

Exemple 4 : Composition selon l'invention

Un exemple de formulation comprenant le principe actif selon l'invention sous forme d'une crème visage anti-rides décontractante est présenté dans le tableau 3 suivant.

[Tableau 3]

| | Ingrédients | % |
|---|---|---|
| A1 | Eau Purifiée | qsp 100 |
| | Conservateur | qs |
| | Butylène Glycol | 3,00 |
| A2 | Glycérine | 2,00 |
| | Gomme Xanthane | 0,20 |
| B | Cetyl Alcohol & Glyceryl Stéarate & PEG-75 Stéarate & Ceteth-20 & Steareth-20 | 4,00 |
| | Squalane | 4,00 |
| | Undecane & Tridecane | 5,00 |
| | Jojoba Esters & Jojoba Alcohol & Propanediol & Tocopherol | 4,00 |
| | Isocetyl Stearoyl Stéarate | 5,00 |
| | Dimethicone | 3,00 |
| C | Polymethylsilsesquioxane | 4,00 |
| D | Hydroxyethyl Acrylate/Sodium Acryloyldimethyl Taurate Copolymer | 0,50 |
| E | Parfum (Fragrance) | 0,50 |
| F | **Principe Actif Invention** | **2,00** |
| G | Solution Soude 28% | qsp pH |

La composition de l'exemple 4 peut notamment être obtenue par le procédé suivant :

- Ajouter A2 dans A1 sous agitation modérée, et chauffer à 80°C.
- Placer B sous agitation et chauffer à 80°C.
- Emulsionner B dans A sous agitation cisaillante pendant 10 minutes.
- A 40°C, sous agitation douce, ajouter C, D, E et F.
- Ajuster le pH à 5,2 - 5,6 avec G.

La composition est alors sous forme d'une émulsion souple, brillante de couleur blanc cassé, de pH égale 5,4 et une viscosité (C/5rpm) égale à 24 000cP, s'étalant facilement sur la peau et laissant un fini doux et confortable.

Exemple 5 : Composition selon l'invention

Un exemple de formulation comprenant le principe actif selon l'invention sous forme d'un primer lissant est présenté dans le tableau 4 suivant.

[Tableau 4]

| | Ingrédients | % |
|---|---|---|
| A1 | Eau Purifiée | qsp 100 |
| | Conservateur | qs |
| | Butylène Glycol | 3,00 |
| A2 | Glycérine | 2,00 |
| | Sclerotium Gum & Xanthan Gum | 0,50 |

(suite)

| | Ingrédients | % |
|---|---|---|
| B | Disodium Cetearyl Sulfosuccinate | 1,00 |
| | Undecane & Tridecane & Tocopherol | 5,00 |
| | Dimethicone, 350 cSt | 4,00 |
| | Dimethicone, 100 cSt | 6,00 |
| | Dimethicone & Dimethicone Crosspolymer | 10,00 |
| C | Polyacrylate Crosspolymer-6 | 0,50 |
| D | Mica | 1,50 |
| | Kaolin | 1,50 |
| E | CI 77891 (Titanium Dioxide) & Mica & CI 77491 (Iron Oxides) | 0,20 |
| F | **Principe Actif Invention** | **2,00** |

La composition de l'exemple 5 peut notamment être obtenue par le procédé suivant :

- Ajouter A2 dans A1 sous agitation modérée, et chauffer à 80°C.
- Placer B sous agitation jusqu'à homogénéité et chauffer à 80°C.
- Emulsionner B dans A sous agitation cisaillante pendant 10min.
- A 40°C, ajouter C puis D, E et F sous agitation modérée.

La composition est alors sous forme d'une émulsion souple, de couleur pêche, préparant la peau à l'application de maquillage sans défaut. Sa texture moelleuse est agréable à appliquer et lisse les imperfections pour un fini naturel.

**Evaluation de l'efficacité du principe actif selon l'invention**

Essai 1 - Etude de la fraction active du principe actif selon l'invention

L'objectif de cette étude est d'identifier la fraction active du principe actif de l'invention. Celui-ci est riche en cendres minérales (au moins 25% de l'actif), pour réaliser cette étude, l'efficacité du principe actif selon l'invention est comparée à celle de l'extrait constituant le principe actif de l'invention déminéralisé, par électrodialyse. On obtient alors un produit dépourvu de 96% des sels minéraux présent dans l'extrait initial, ci-après nommé « extrait déminéralisé ».

L'objectif de cette étude est de comparer la capacité du principe actif selon l'invention et de l'extrait déminéralisé à moduler l'expression du gène OXTR (Oxytocin receptor) impliqué dans les voies du bien-être. Cette étude est réalisée par PCR quantitative sur des kératinocytes humains normaux. Les résultats sont présentés dans le tableau 5, ci-après.

[Tableau 5]

| | Expression de OXTR (%) | Capacité à booster l'expression de OXTR (%) |
|---|---|---|
| Témoin | 100 | |
| INVENTION 0,50% | 666 ± 19 | **+566** |
| Extrait déminéralisé 0,50% | 98 ± 8 | **+0** |

Le principe actif selon l'invention augmente l'expression du gène OXTR de +566% alors que l'extrait déminéralisé ne modifie pas l'expression dudit gène. Ainsi, ce sont les minéraux présents dans l'extrait qui sont responsables de la stimulation de l'expression de OXTR.

Essai 2 - Etude du principe actif selon l'invention sur la contraction musculaire

L'objectif de cette étude est d'évaluer la capacité du principe actif selon l'invention à réduire la fréquence de contractions de cellules musculaires et par conséquent d'évaluer l'effet myorelaxant.

Cette étude a été réalisée sur un modèle de neurones sensitifs humains dérivés de cellules hiPS (human induced Pluripotent Stem cells) en co-culture avec des cellules musculaires humaines. Le nombre de contractions musculaires a été suivi et enregistré à l'aide d'un microscope équipé d'une caméra.

Le protocole est le suivant. Des motoneurones issus de cellules hiPS sont mis en culture avec des cellules musculaires humaines. Le milieu de culture a été changé régulièrement.

Le principe actif selon l'invention à 0,5%, 1,0% ou 2,0% est ajouté dans les puits de culture. Un témoin positif présentant une activité myorelaxante, a été utilisé à 2 $\mu$M. Le comptage du nombre de contractions est réalisé après 6 heures.

Le nombre de contractions est déterminé pendant 1 minute et les résultats sont exprimés en pourcentage selon la formule suivante.

[Math 1]

$$\text{Pourcentage de contractions} = \frac{\text{Nombre de contractions t}_{6h} - \text{Nombre de contractions à t}_0}{\text{Nombre de contractions t}_0} \times 100$$

Les résultats sont présentés dans le tableau 6, ci-après.

[Tableau 6]

|  | Pourcentage de contractions (%) | Capacité à réduire le pourcentage de contractions (%) |
|---|---|---|
| Témoin | 8 |  |
| Témoin positif 2$\mu$M | -21 | **-29** |
| INVENTION 0,5% | -21 | **-29** |
| INVENTION 1,0% | -25 | **-33** |
| INVENTION 2,0% | -31 | **-39** |

Dès la dose de 0,5%, le principe actif selon l'invention inhibe significativement les contractions musculaires sur un modèle de co-culture nerf-muscle, effet dose observé jusqu'à 2%. Ainsi, le principe actif selon l'invention présente une activité myorelaxante.

Essai 3 - Etude du principe actif selon l'invention sur les médiateurs du bien-être

*Essai 3a - Etude sur les voies ocytocine et endocannabinoïdes.*

L'objectif de cette étude est d'évaluer la capacité du principe actif selon l'invention à stimuler les médiateurs et récepteurs du bien-être, notamment impliqués dans les voies ocytocine et endocannabinoïdes.

L'étude a été réalisée via l'analyse de :

- l'expression de PIEZO1, OXT et OXTR par PCR quantitative sur kératinocytes humains issus de donneurs d'âge médian (54 ans) ;
- la synthèse d'ocytocine et du récepteur aux cannabinoïdes de type 1 (CB1R), par dosage ELISA et marquage par immunohistofluorescence, sur explants de peau humaine issus de donneurs d'âge médian 38 ans.

Le protocole de l'étude de l'expression de PIEZO1, OXT et OXTR est le suivant. Les kératinocytes humains sont ensemencés et incubés, puis les cellules sont traitées avec du milieu de culture contenant le principe actif selon l'invention à 0,25% ou 0,50% (V/V). Enfin, les kératinocytes sont récupérés et les ARN totaux extraits, puis analysés par la technique de PCR quantitative.

Le protocole de l'étude de la synthèse d'ocytocine et du récepteur aux cannabinoïdes de type 1 (CB1R) est le suivant. Des explants de peau humaine ont été réalisés et maintenus en survie dans un milieu de culture adapté, puis les explants sont traités tous les jours en systémique avec le principe actif selon l'invention à 0,5% et 1,0% (V/V) pendant plusieurs jours. Les sous-nageants sont récupérés et dosés par un test ELISA afin de mesurer la synthèse de l'ocytocine.

Concernant, l'étude sur le récepteur aux cannabinoïdes de type 1 (CB1R), les explants sont récupérés, fixés, déshydratés et inclus en paraffine. Des coupes sont ensuite réalisées, puis marquées avec un anticorps primaire anti-CB1R et un anticorps secondaire, et enfin, visualisées par microscope.

Les résultats de l'expression de PIEZO1, OXT et OXTR sont présentés dans le tableau 7 ci-après.

[Tableau 7]

| | Expression (%) | Capacité à augmenter l'expression (%) |
|---|---|---|
| **PIEZO1** | | |
| Témoin | 100 | |
| INVENTION 0,25% | 135 | **+35** |
| INVENTION 0,50% | 187 | **+87** |
| **OXT** | | |
| Témoin | 100 | |
| INVENTION 0,25% | 141 | **+41** |
| INVENTION 0,50% | 275 | **+175** |
| **OXTR** | | |
| Témoin | 100 | |
| INVENTION 0,25% | 239 | **+139** |
| INVENTION 0,50% | 651 | **+551** |

Testé à 0,5% sur des kératinocytes humains, le principe actif selon l'invention augmente significativement l'expression de PIEZO1, de l'ocytocine et de son récepteur de respectivement 87%, 175% et 551%.

Les résultats de la synthèse d'ocytocine sont présentés dans le tableau 8, ci-après.

[Tableau 8]

| | Synthèse d'ocytocine (pg/mL) | Capacité à augmenter la synthèse d'ocytocine (%) |
|---|---|---|
| Témoin | 30 | |
| INVENTION 0,5% | 41 | **+37** |
| INVENTION 1,0% | 49 | **+63** |

Testé à 1,0% sur des explants de peau humaine, le principe actif selon l'invention augmente significativement la synthèse d'ocytocine de 63%.

Les résultats de la synthèse de CB1R sont présentés dans le tableau 9, ci-après.

[Tableau 9]

| | Synthèse de CB1R (UA) | Capacité à augmenter la synthèse de CB1R (%) |
|---|---|---|
| Témoin | 24 | |
| INVENTION 0,5% | 33 | **+38** |
| INVENTION 1,0% | 35 | **+46** |

Testé à 1,0% sur des explants de peau humaine, le principe actif selon l'invention augmente significativement la synthèse du récepteur aux cannabinoïdes de type 1 de 46%.

Testé sur kératinocytes et explants humains, le principe actif selon l'invention agit sur les voies ocytocine et endocannabinoïdes. Il permet ainsi de stimuler les médiateurs et récepteurs du bien-être.

*Essai 3b - Etude de la prolifération et de la différenciation épidermique*

Au niveau de la peau, les effets du stress psychologique se traduisent par une perturbation du processus de prolifération et une altération de la fonction barrière. Il a été récemment démontré que l'activation de la voie des endocannabinoïdes, plus particulièrement le récepteur de type 1 (CB1R), ainsi que la voie de l'ocytocine stimulent la prolifération épidermique. De plus, la signalisation du CB1R est nécessaire à une différenciation optimale de l'épiderme et au maintien d'une fonction barrière intègre. Dans ce contexte, l'inventeur a évalué la capacité du principe actif selon l'invention à stimuler la prolifération des kératinocytes et la différenciation épidermique.

L'objectif de cette étude est d'évaluer la capacité du principe actif selon l'invention à stimuler la prolifération et la différenciation kératinocytaire.

Le rôle de protection de l'épiderme face à l'environnement extérieur est assuré par les kératinocytes qui vont d'une part proliférer et contribuer au renouvellement du tissu, et d'autre part se différencier et entrer dans différents stades de maturation pour générer une fonction barrière efficace.

La filaggrine est un élément essentiel à la fonction barrière épidermique car elle contribue à la rigidité du tissu ainsi qu'à son hydratation.

Le protocole est identique à celui décrit dans l'étude de la synthèse du récepteur aux cannabinoïdes de type 1 (Essai 3a), à la différence que l'anticorps primaire est un anticorps anti-filaggrine.

Les résultats de la prolifération cellulaire sont présentés dans le tableau 10, ci-après.

[Tableau 10]

|  | Prolifération (UA) | Capacité à augmenter la prolifération (%) |
|---|---|---|
| Témoin | 81 380 |  |
| INVENTION 0,25% | 130 823 | **+61** |
| INVENTION 0,50% | 137 363 | **+69** |

Testé à 0,5% sur kératinocytes humains, le principe actif selon l'invention augmente significativement leur prolifération de 69%.

Les résultats de la synthèse de la filaggrine sont présentés dans le tableau 11, ci-après.

[Tableau 11]

|  | Synthèse de filaggrine (UA) | Capacité à augmenter la synthèse de filaggrine (%) |
|---|---|---|
| Témoin | 789 |  |
| INVENTION 0,5% | 974 | **+23** |
| INVENTION 1,0% | 990 | **+25** |

Testé à 1,0% sur explants de peau humaine, le principe actif selon l'invention augmente significativement la synthèse de filaggrine de 25%.

Testé sur kératinocytes et explants humains, le principe actif selon l'invention favorise la prolifération et la différenciation, permettant ainsi de réguler l'homéostasie épidermique.

*Essai 4 - Etude in vivo de l'effet lissant du principe actif selon l'invention*

L'objectif de cette étude est d'évaluer *in vivo* l'effet lissant du principe actif selon l'invention formulé à 2% en émulsion après 5 et 28 jours d'application biquotidienne sur l'ensemble du visage, en comparaison à une formule placebo.

L'étude a été menée sur un panel caucasien, comprenant un groupe jeune présentant des premières rides d'expression. Le groupe jeune est composé de 16 volontaires sains, de sexe féminin (âge moyen 35 ans) comparé au groupe placebo de 16 volontaires sains, de sexe féminin (âge moyen 35ans). L'ensemble des sujets a été sélectionné selon un niveau de stress modéré à important d'après le questionnaire Perceived Stress Scale (PSS10).

L'effet lissant est évalué à l'aide d'un appareil de projection de franges dédié à la mesure 3D du relief cutané. Le paramètre retenu pour cette étude est le paramètre de rugosité Rz qui traduit la profondeur des rides. Plus ce paramètre est élevé, plus la profondeur des sillons est importante.

Les résultats sont présentés dans le tableau 12, ci-après.

[Tableau 12]

| | Variation / J0 (%) | | | |
|---|---|---|---|---|
| | Front | Patte d'oie | Glabelle | Sillon naso-génien |
| Placebo | -0,4 | -1,2 | -0,2 | -0,1 |
| INVENTION | -3,6 | 0,9 | -5,3 | -3,9 |
| Variation / Placebo (%) | -3,2 | 2,1 | -5,1 | -3,8 |

Le principe actif selon l'invention favorise le lissage rapide (en seulement 5 jours d'application biquotidienne sur l'ensemble du visage) des premières rides d'expression de volontaires caucasiens jeunes en comparaison au placebo, en particulier au niveau du front, de la glabelle et du sillon nasogénien.

Ainsi, les premières rides d'expression des volontaires jeunes sont lissées rapidement.

Essai 5 - Etude *in vivo* sur l'effet « soft focus » au niveau du visage

Le protocole d'étude est identique à celui de l'essai 4. Des photographies standardisées du visage, sans expression, ont été réalisées de face et de côté.

Les photographies ont été retouchées par des experts en analyse d'image à l'aide de logiciels informatiques afin de lisser le visage en ciblant l'aspect des rides. L'évaluation a été réalisée par un panel d'évaluateurs naïfs en comparaison à des images réelles. Les évaluateurs ont jugé l'amélioration globale de l'aspect du visage du volontaire en comparant l'image de référence, prise avant le début du traitement et les images à évaluer : l'image retouchée à l'aide de logiciels, la photographie à J5 après application, la photographie à J28 après application.

Les résultats sont présentés dans le tableau 13, ci-après.

[Tableau 13]

| | Amélioration moyenne (UA) | |
|---|---|---|
| | Placebo | INVENTION 2% |
| Image retouchée | 1,41 | 1,40 |
| J5 | 0,81 | 1,08 |
| J28 | 0,72 | 1,14 |

Dès 5 jours d'application biquotidienne, le principe actif selon l'invention à 2% permet aux volontaires caucasiens jeunes d'obtenir un résultat visible se rapprochant de celui obtenu avec une retouche photo. Cet effet s'intensifie après 28 jours de traitement, moment où la note attribuée à l'image retouchée n'est plus significativement différente de celle attribuée à l'image après application.

Essai 6 - Etude *in vivo* sur le bien-être du principe actif selon l'invention

Le protocole d'étude est identique à celui de l'essai 4, y compris le panel jeune caucasien. Parmi les 16 volontaires inclus dans ce groupe, le marquage d'ocytocine a été réalisé sur 12 sujets (âge moyen 36 ans). Le protocole d'étude a également été réalisé sur un panel mature avec rides d'expression installées. Ce panel est composé de 17 sujets (âge moyen 57 ans). Parmi ces volontaires, le marquage a été réalisé sur 12 sujets (âge moyen 58 ans). La quantité d'ocytocine produite au niveau des avant-bras a été mesurée par immunocytofluorescence. La synthèse d'ocytocine est proportionnelle à l'intensité de fluorescence. Plus la fluorescence est importante, plus la synthèse d'ocytocine est élevée.

Les résultats sont présentés dans le tableau 14 (panel jeune) et 15 (panel mature), ci-après.

[Tableau 14]

| | Variation / J0 (%) | | Variation / Placebo (%) |
|---|---|---|---|
| | Placebo | INVENTION | |
| J5 | 5,1 | 70,3 | 65 |

(suite)

| | Variation / J0 (%) | | Variation / Placebo (%) |
|---|---|---|---|
| | Placebo | INVENTION | |
| J28 | -10,6 | 63,6 | 74 |

[Tableau 15]

| | Variation / J0 (%) | | Variation / Placebo (%) |
|---|---|---|---|
| | Placebo | INVENTION | |
| J5 | 16,3 | 153,3 | 137 |
| J28 | 23,4 | 162,4 | 139 |

Dès 5 jours d'application biquotidienne et en comparaison au placebo, le principe actif selon l'invention à 2% augmente la production d'ocytocine chez les volontaires jeunes de 65% et chez les volontaires matures de 137%. Ces effets s'intensifient encore après 28 jours de traitement avec une augmentation de la production d'ocytocine de 74% chez les volontaires jeunes et de 139% chez les volontaires matures.

L'effet du principe actif selon l'invention sur la perception du stress a également été déterminé à l'aide d'un questionnaire d'évaluation. Pour cette étude, l'échelle *Perceived Stress Scale* (PSS10) a été employée. Elle se base sur l'approche transactionnelle du stress afin d'appréhender les mécanismes psycho-cognitifs de ce dernier.

Les résultats correspondant à la perception du stress des sujets ayant participé à l'évaluation de la formule placebo ou de la formule comprenant le principe actif selon l'invention formulé à 2% pendant 28 jours, sont présentés dans les tableaux 16 (panel jeune) et 17 (panel mature), ci-après.

[Tableau 16]

| | Variation / J0 | Variation / Placebo (%) |
|---|---|---|
| Placebo | -10,4 | |
| INVENTION | -15,2 | -4,9 |

[Tableau 17]

| | Variation / J0 | Variation / Placebo (%) |
|---|---|---|
| Placebo | -7,6 | |
| INVENTION | -16,6 | -9,0 |

Après 28 jours d'utilisation biquotidienne du principe actif selon l'invention à 2%, l'ensemble des sujets jeunes ou matures notent une réduction du stress perçu dans leur vie quotidienne. Cet effet a été observé :

- chez 75% des sujets jeunes ayant utilisé le principe actif selon l'invention contre 56% des sujets ayant appliqué la formule placebo ;
- chez 71% des sujets matures ayant utilisé le principe actif selon l'invention contre 59% des sujets ayant appliqué la formule placebo.

En conclusion, dès 5 jours d'application biquotidienne par des volontaires jeunes et matures, le principe actif selon l'invention formulé à 2% en émulsion stimule la production de l'hormone du bonheur ocytocine. Ces effets se prolongent et s'intensifient après 28 jours de traitement.

De plus, en comparaison à un traitement avec une émulsion placebo, le principe actif selon l'invention permet aux volontaires jeunes et matures de se sentir mieux dans leur peau et ainsi de moins percevoir le stress du quotidien.

Essai 7 - Etude *in vivo* sur l'éclat du teint de la peau

Le protocole d'étude est identique à celui de l'essai 5, y compris le panel caucasien.
L'effet du principe actif selon l'invention a été évalué au niveau du visage après 5 et 28 jours d'application biquotidienne en analysant l'éclat du teint sur photographies numériques.
Les résultats sont présentés dans le tableau 18 ci-après.

[Tableau 18]

| | Variation / Placebo (%) | | | |
|---|---|---|---|---|
| | Rayonnement | Couleur rose | Couleur olive | Fatigue des yeux |
| J5 | 5,6 | 28,8 | -11,0 | -15,3 |
| J28 | 11,5 | 34,7 | -12,7 | -16,3 |

En seulement 5 jours d'application, le principe actif selon l'invention formulé à 2% améliore significativement les paramètres caractéristiques de l'éclat du teint des volontaires caucasiens jeunes. En effet, le principe actif selon l'invention rend le teint plus lumineux, plus frais et améliore l'aspect bonne mine en réduisant la couleur olive et la fatigue des yeux (effets significatifs par rapport au placebo).
Ces effets s'améliorent encore après 28 jours de traitement avec une augmentation de 11,5% du rayonnement de la peau et de 34,7% de la couleur rose. On observe également une diminution de la couleur olive de 12,7% ainsi que de l'état de fatigue des yeux de 16,3% chez 50% des volontaires.
Dès 5 jours d'application biquotidienne par des volontaires caucasiens jeunes, le principe actif selon l'invention à 2% améliore l'éclat du teint de la peau.
Cet effet se maintient après 28 jours d'application.
En conclusion, le principe actif selon l'invention permet de lutter contre le stress, notamment ses effets délétères, en agissant sur la voie ocytocine et endocannabinoïde, en particulier les récepteurs et médiateurs du bien-être, ce qui se traduit par une amélioration de l'éclat du teint de la peau, et donc permet de lutter contre le stress cutané.

**Revendications**

1. Principe actif comprenant au moins un extrait de *Nasturtium officinale,* pour son utilisation pour lutter contre le stress, en application topique, **caractérisé en ce que** l'extrait de *Nasturtium officinale* comprend des minéraux.

2. Principe actif pour son utilisation selon la revendication précédente, pour lutter contre les effets cutanés du stress.

3. Principe actif pour son utilisation selon l'une des revendications précédentes, **caractérisé en ce que** ledit principe actif présente un effet myorelaxant cutané.

4. Principe actif pour son utilisation selon l'une des revendications précédentes, **caractérisé en ce que** ledit principe actif stimule les médiateurs et récepteurs du bien-être.

5. Principe actif pour son utilisation selon la revendication précédente, **caractérisé en ce que** ledit principe actif augmente la sécrétion d'ocytocine cutanée (OXT).

6. Principe actif pour son utilisation selon l'une des revendications précédentes, **caractérisé en ce que** ledit principe actif augmente l'expression du récepteur PIEZO1.

7. Principe actif pour son utilisation selon l'une des revendications précédentes, **caractérisé en ce que** ledit principe actif augmente l'expression du récepteur OXTR.

8. Principe actif pour son utilisation selon l'une des revendications précédentes, **caractérisé en ce que** ledit principe actif augmente la synthèse du récepteur aux cannabinoïdes de type 1 (CB1R).

9. Principe actif pour son utilisation selon l'une des revendications précédentes, **caractérisé en ce que** l'extrait de *Nasturtium officinale* est un hydrolysat de *Nasturtium officinale.*

**10.** Principe actif pour son utilisation selon la revendication précédente, **caractérisé en ce que** l'hydrolysat de *Nasturtium officinale* est un hydrolysat enzymatique de *Nasturtium officinale.*

**11.** Principe actif pour son utilisation selon l'une des revendications précédentes, **caractérisé en ce que** les minéraux représentent au moins 25% en poids du poids total de l'extrait

**12.** Principe actif pour son utilisation selon l'une des revendications précédentes, **caractérisé en ce que** l'extrait comprend au moins un minéral choisi parmi les chlorures, le soufre, le sodium, le potassium, le magnésium, le phosphore, le calcium, et leurs combinaisons.

**13.** Utilisation cosmétique d'un principe actif selon l'une des revendications précédentes, pour améliorer l'éclat du teint.

**14.** Utilisation cosmétique d'un principe actif selon l'une des revendications 1 à 12, pour un effet lissant immédiat.

**RAPPORT DE RECHERCHE EUROPEENNE**

Europäisches Patentamt
European Patent Office
Office européen des brevets

Numéro de la demande

EP 24 17 9182

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | FR 2 834 462 B1 (SILAB SA [FR]) 12 mars 2004 (2004-03-12) * page 7, ligne 25 - page 8, ligne 7 * ----- | 1-14 | INV. A61P17/00 A61K8/9789 A61K36/31 |
| X | Anonymous: "NASTURTIUM OFFICINALE EXTRACT Cosmetic Ingredient (INCI)", , 20 janvier 2022 (2022-01-20), XP093100083, Extrait de l'Internet: URL:https://web.archive.org/web/2022012021 1135/https://cosmetics.specialchem.com/inc i-ingredients/nasturtium-officinale-extrac t [extrait le 2023-11-10] | 1-10,12, 14 | |
| Y | * le document en entier * ----- | 11 | |
| X | HARO G ET AL: ""EVALUATION OF ANTIOXIDANT ACTIVITY AND MINERALS VALUE FROM WATERCRESS (Nasturtium officinale R.Br.) "", RASAYAN JOURNAL OF CHEMISTRY , 1 janvier 2018 (2018-01-01), XP093100284, India ISSN: 0974-1496, DOI: 10.7324/RJC.2018.1112011 Extrait de l'Internet: URL:https://rasayanjournal.co.in/admin/php /upload/341_pdf.pdf | 1-10,12 | |
| Y | * le document en entier * * pages 134-235; figure 2; tableau 3 * ----- -/-- | 11 | |

**DOMAINES TECHNIQUES RECHERCHES (IPC)**

A61P
A61K
A61Q

Le présent rapport a été établi pour toutes les revendications

| Lieu de la recherche | Date d'achèvement de la recherche | Examinateur |
|---|---|---|
| Munich | 9 septembre 2024 | Fayos, Cécile |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

page 1 de 3

Europäisches
Patentamt

European
Patent Office

Office européen
des brevets

**RAPPORT DE RECHERCHE EUROPEENNE**

Numéro de la demande

EP 24 17 9182

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | Solabia: "CRESSATINE", , 1 juin 2017 (2017-06-01), XP093100281, Extrait de l'Internet: URL:https://www.biogreenscience.com/clinical-bio-sifvlevio/asset/cressatine-sifvlevio.pdf [extrait le 2023-11-10] | 1-10,12 | |
| Y | * le document en entier * * THE ACTIVE 5 "Watercress"; page 8 * | 11 | |
| X | Farmalabor: "TECHNICAL DATASHEET 0855 NASTURTIUM OFFICINALE LIQUID EXTRACT", , 3 octobre 2022 (2022-10-03), XP093100524, Extrait de l'Internet: URL:https://web.archive.org/web/20221003165740if_/https://materie-prime.farmalabor.it/schede/117312.PDF [extrait le 2023-11-13] | 1-10,12 | |
| Y | * le document en entier * & Anonymous: "Wayback Machine", , 1 octobre 2022 (2022-10-01), XP093100525, Extrait de l'Internet: URL:https://web.archive.org/web/20221001000000*/https://materie-prime.farmalabor.it/schede/117312.PDF [extrait le 2023-11-13] * le document en entier * | 11 | DOMAINES TECHNIQUES RECHERCHES (IPC) |
| X | "Spotvest Anti-Brown Spot Radiance", GNPD, MINTEL, 1 octobre 2015 (2015-10-01), XP002770956, | 1-10, 12-14 | |
| Y | * le document en entier * | 11 | |

-/--

Le présent rapport a été établi pour toutes les revendications

| Lieu de la recherche | Date d'achèvement de la recherche | Examinateur |
|---|---|---|
| Munich | 9 septembre 2024 | Fayos, Cécile |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.............................................................................
& : membre de la même famille, document correspondant

EPO FORM 1503 03.82 (P04C02)

page 2 de 3

**EP 4 470 610 A1**

Europäisches Patentamt
European Patent Office
Office européen des brevets

# RAPPORT DE RECHERCHE EUROPEENNE

Numéro de la demande

EP 24 17 9182

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | KLIMEK-SZCZYKUTOWICZ MARTA ET AL: "Chemical composition, traditional and professional use in medicine, application in environmental protection, position in food and cosmetics industries, and biotechnological studies ofNasturtium officinale(watercress) - a review", FITOTERAPIA, IDB HOLDING, MILAN, IT, vol. 129, 28 mai 2018 (2018-05-28), pages 283-292, XP085441963, ISSN: 0367-326X, DOI: 10.1016/J.FITOTE.2018.05.031 | 1-10, 12-14 | |
| Y | * le document en entier * ----- | 11 | |
| | | | **DOMAINES TECHNIQUES RECHERCHES (IPC)** |

Le présent rapport a été établi pour toutes les revendications

| Lieu de la recherche | Date d'achèvement de la recherche | Examinateur |
|---|---|---|
| Munich | 9 septembre 2024 | Fayos, Cécile |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

page 3 de 3

## ANNEXE AU RAPPORT DE RECHERCHE EUROPEENNE
## RELATIF A LA DEMANDE DE BREVET EUROPEEN NO.

EP 24 17 9182

La présente annexe indique les membres de la famille de brevets relatifs aux documents  brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

09-09-2024

| Document brevet cité au rapport de recherche | Date de publication | Membre(s) de la famille de brevet(s) | Date de publication |
|---|---|---|---|
| FR 2834462          B1 | 12-03-2004 | AUCUN | |

EPO FORM P0460

Pour tout renseignement concernant cette annexe : voir Journal Officiel de l'Office européen des brevets, No.12/82

## RÉFÉRENCES CITÉES DANS LA DESCRIPTION

**Documents brevets cités dans la description**

- FR 3079145

**Littérature non-brevet citée dans la description**

- **DUBOIS M. et al.** *Analytical chemistry,* 1956, vol. 28 (3), 350-356

- **P. K. SMITH et al.** Measurement of protein using bicinchoninic acid. *Anal. Biochem.,* 1985, vol. 150 (1), 76